# EUROPEAN PATENT APPLICATION

(11) **EP 2 127 682 A1**
(43) Date of publication of application: **02.12.2009**
(21) Application number: 08157046.7
(22) Date of filing: 28.05.2008
(51) Int. Cl.: A61K 49/04

(54) **X-ray contrast agent based on bismuth oxide-nanoparticles**

(71) Applicant: Deutsches Krebsforschungszentrum, 69120 Heidelberg (DE); Merck Patent GmbH, 64293 Darmstadt (DE)
(72) Inventor: Huppert, Jochen, 66265, Holz (DE); Kiessling, Fabian c/o Universität Aachen (RWTH), Pauwelsstr. 20, 52074 Aachen (DE); Jayapaul, Jabadurai, 69120 Heidelberg (DE); Kübelbeck, Armin, 64625, Bensheim (DE); Larbig, Gregor, 63571, Gelnhausen (DE)
(74) Representative: Liesegang, Eva

(57) **Abstract**

An X-ray contrast agent comprising a Bi₂O₃-nanoparticle covered with a cover layer for stabilizing and/or functionalizing the contrast agent is shown. Further described is a set of such contrast agents, as well as a method for their manufacture.

## Description

The present invention relates to an X-ray contrast agent and its manufacturing method as well as a set or platform comprising contrast agents. Also, the present invention relates to the use of a contrast agent in a dual-energy computed tomography (dual-energy CT).

In X-ray imaging, contrast agents can be used to improve the visibility of internal body structures or specific body functions. Herein, the term "X-ray imaging" relates to any type of medical imaging using X-rays and covers both, traditional X-ray imaging as well as CT-imaging. X-ray contrast agents contain an agent that has a higher X-ray absorption than normal body tissue. For example, in a normal X-ray image blood vessels cannot be seen, since the blood has a similar absorption coefficient as the surrounding tissue. If an X-ray contrast agent is injected into a vessel, due to the contrast agent in the blood, the lumen of blood vessels and organs of the body can be visualized.

In the medical imaging of vessels, which is known as angiography, usually low molecular iodine containing contrast agents are used. However, this type of contrast agent only allows for a very narrow time window for taking the X-ray image after its injection. Also, due to extravasation of the contrast agent from the vessels, the contrast between smaller diameter vessels and the interstitium tends to be blurred.

Another X-ray contrast agent currently used is barium sulphate (BaSO₄), which due to its toxicity however can only be used in pre-clinical studies and in particular, in angiography of small animals.

In a recent article "X-ray computer-tomography imaging agent based on long-circulating bismuth sulphide nanoparticles", O. Rabin et al., Nature Materials, Vol. 5, Feb. 2006, pp. 118-122, the use of a polymer-coated Bi₂S₃-nanoparticle as an X-ray contrast agent is described. The potential for toxicity, however, remains a factor determining its specific applicability.

With the prior art contrast agents, it is difficult to provide for an X-ray imaging that is specific for a certain target region or biological functions. It is an object of the present invention to provide an X-ray contrast agent that allows for a target and/or function-specific X-ray imaging.

This object is achieved by an X-ray contrast agent according to claim 1, by a set of X-ray agents according to claim 7 and by a method of making an X-ray contrast agent according to claim 8. Preferable embodiments are defined in the depending claims.

According to the invention, a new X-ray contrast agent is provided which comprises bismuth oxide (Bi₂O₃) nanoparticles covered with a cover layer for stabilizing and/or functionalizing the contrast agent.

This new contrast agent is found to have little or no nephrotoxicity and does not interfere with the thyroid hormone functions. Also, as will be shown below, the use of bismuth oxide nanoparticles allows for a controlled and reproducible variation of the size and surface of the particle, which in turn allows for a target area and function-specific imaging. In particular, this allows that different predetermined metabolic pathways become accessible. At the same time, due to the high electron density of the bismuth, the X-ray absorption is even higher than that of barium or iodine, which allows for an enhanced contrast.

Preferably, the bismuth oxide nanoparticles are spherical.

Preferably, the diameter of the Bi₂O₃-nanoparticles is larger than 20 nm, more preferably larger than 30 nm, and most preferably larger than 40 nm. As will be shown below, such size and even larger nanoparticle sizes can be easily obtained for Bi₂O₃-nanoparticles. With this size of nanoparticles, the contrast agent will be much less prone to extravasation from the vessel. This kind of contrast agents, which remain within the intravascular compartment for a substantially longer time than ordinary contrast agents, are often called blood-pool agents for obvious reasons.

It is noted that blood- pool agents have a number of important advantages. In anatomic imaging, blood- pool agents allow a reduction of dose, since they allow to avoid the re-injection and increase of the temporal imaging window. Also, blood- pool agents allow for a better definition of small vessels and for an easier detection of bleeding areas. In functional imaging, blood- pool agents allow for a quantification of blood volume, tissue perfusion and permeability. Accordingly, blood- pool agents are very useful for a number of reasons, and Bi₂O₃-nanoparticles have been found to be a very promising basis for a new platform of blood- pool agents.

In a preferred embodiment, the cover layer comprises a stabilizing layer suitable for increasing the inertness and decreasing the solubility of the bismuth oxide nanoparticle, and a functionalizing layer formed on top of the stabilizing layer for functionalizing the contrast agent. An example of a suitable stabilizing layer is a SiO₂ layer, which can be grown on top of the bismuth oxide nanoparticles, as will be described below. However, other stabilizing layers can be used as long as they have sufficient inertness, such as to stabilize the nanoparticles.

In a preferred embodiment, the functionalizing layer may comprise silane molecules bound to the stabilizing layer. In the present disclosure, the term "silane molecule" covers any type of silane containing molecule, including aminosilanes, glycidoxysilanes and mercaptosilanes. In particular, mono-, di- and/or tri-alkoxysilane molecules can be bound to the stabilizing layer to form the functionalizing layer. By choosing specific silane molecules, the surface of the contrast agent can be functionalized, such that the contrast agent when administered to the blood will enter specific metabolic pathways.

Further, in a preferred embodiment the functionalizing layer comprises polar polymers attached to the stabilizing layer. In this regard, reactive silane molecules which have been bound to the stabilizing layer before help to attach the polymers. Then, by choosing the type and/or length of the polymer, the desired functionality can be achieved.

However, the present invention is by no means restricted to SiO₂ stabilizing layers, nor is it restricted to using silane molecules for the functionalization. In some embodiments of the invention, there will not be a clear distinction between a stabilizing and functionalizing layer, as a single cover layer can have stabilizing and functionalizing properties at the same time.

In a preferred embodiment, the Bi₂O₃-nanoparticles can be coated with a wide range of monomeric species, including amino acids, α-hydroacids (citric acid, tartatic acid, gluconic acid), hydroxamate (arginine hydroxamate), or dimercaptosuccinic acid (DMSA).

In another preferred embodiment, a polymeric coating can be used. Particularly suitable polymeric coatings are formed by dextran, carboxymethylated dextran, carboxydextran, starch, polyethylenglycol (PEG), arabinogalactan, glycaminoglycan, organic siloxane, and sulfonated styrene-divinylbenzene. Also, the Bi₂O₃-nanoparticles can be coated using dendrimers like carboxylated polyamino amine (PAMAM).

It is found that the Bi₂O₃-nanoparticles can in fact be coated with a large number of different materials, and this is why it is very suitable for functionalization. To give one example, a Bi₂O₃-nanoparticle has been found to be suitable for coating with PEG, which will reduce or slow down the absorption of the particle by the reticulo-endothial system, such as the liver. This allows for a prolonged circulation time of the particle, such that longer scanning intervals are possible.

Further, by using suitable coatings, it becomes possible to visualize plaques. Also, by using a suitable coating, activated macrophages can be targeted.

In a second aspect, the invention relates to a set of X-ray contrast agents, in which each of the contrast agents is a contrast agent according to one of the embodiments described above. In this set, each of the individual contrast agents is specified for use for one or a group of different target areas or metabolic pathways, where the specification is based on the size of the nanoparticle and/or the constitution of its cover layer. Herein, the constitution of the cover layer can be defined by a specific choice of functionalizing layer.

The set of X-ray contrast agents thus resembles a platform of contrast agents, which is based on bismuth oxide nanoparticles. This platform can comprise a large variety of X-ray contrast agents specifically adapted for predetermined target areas or body functions. The specific design of the contrast agents constituting this set or platform is made possible by the use of bismuth oxide nanoparticles, which can be reproducibly produced at different predetermined sizes, and due to the choice of the size, the contrast agent will be specifically adapted for certain target areas. Moreover, it has been found that the surface of the bismuth oxide nanoparticles is well suited for a functionalization, by which the specificity of the contrast agent can be further increased.

According to a third aspect, the present invention relates to a method of making an X-ray contrast agent. The method comprises a Bi₂O₃-nanoparticle forming step of forming Bi₂O₃-nanoparticles, and a covering step of forming a cover layer on top of the Bi₂O₃-nanoparticles, wherein said cover layer is suitable for stabilizing and/or functionalizing the contrast agent.

Preferably, the nanoparticle forming step comprises a step of precipitating the Bi₂O₃-nanoparticles from a Bi (NO₃)₃ solution in the presence of a dispersant, such as a PEG dispersant. Such a method of forming Bi₂O₃-nanoparticles is known from "Facile synthesis of monodisperse Bi2O3 nanoparticles", Wei Li, Materials Chemistry and Physics 99 (2006), 174-180. However, in that article the Bi₂O₃-nanoparticles have been considered for entirely different purposes, namely for use as catalysts, electrical ceramics, solid state electrolytes, gas sensors and superconductors.

Precipitating the Bi₂O₃-nanoparticles from a Bi (NO₃)₃-solution has a number of advantages. In particular, this manufacturing process is found to have a high selectivity of the size and size distribution of the nanoparticles according to processing parameters, such as reaction temperature, reaction time, reactant and dispersant concentration. This means that it is easily possible to acquire a variety of different sizes by a suitable adjustment of process parameters, and the control of size and size distribution is found to be sufficiently reproducible. Therefore, in combination with this specific method of manufacture, the Bi₂O₃-nanoparticles represent a very advantageous basis for a new contrast agent platform allowing for target- and function-specific imaging. In addition, the Bi₂O₃-nanoparticle formation step has a satisfactory yield, a good shape uniformity and involves fairly low costs.

Preferably, the covering step comprises a step of growing an inert layer on top of the Bi₂O₃- nanoparticles. This inert layer can for example be a SiO₂-layer, which can be provided on the Bi₂O₃-nanoparticles in a sol-gel-process.

In a preferred embodiment, the step of growing an inert layer comprises the step of forming a SiO₂-layer on top of the Bi₂O₃-nanoparticles by suspensing Bi₂O₃-nanoparticles in an aqueous solution having a pH of 8.5 to 9.5 and adding a solution containing tetraethylorthosilicate.

Preferably, the covering step comprises a step of causing silane molecules to bind with the surface of the inert layer. If the inert layer is a SiO₂-layer, the silane molecules can preferably be caused to bind with the SiO₂-layer by adding silane molecules to the same solution in which the SiO₂-layer has been grown. This has proven to be an efficient and simple way of causing the silane molecules to bind with a SiO₂-layer. This process is preferably conducted such that the silane molecules bind with the surface of the SiO₂-layer at a density of two silane molecules per nm² or more, which has been found to be easily possible with the described method.

Also, the covering step preferably further comprises a step of binding a polar polymer to the cover layer. A suitable polymer could for example be dextrane.

According to a further aspect of the invention, the new X-ray contrast agent based on Bi₂O₃-nanoparticles is used in dual-energy computed tomography. In dual-energy tomography, two X-ray tubes are used which operate at different voltages, such as 140 kV and 80 kV, and with a ratio between the tube currents of for example 1:3. Dual-energy CT offers a more specific tissue characterization and improves the assessment of vascular disease. With two tubes and detectors mounted orthogonally, two spiral acquisitions can run simultaneously, which excludes changes in contrast enhancement or patient movement between acquisitions.

In short, advantages of dual-energy CT are as follows: the dose values remain below legally required levels and close to routine examination protocol, the images are less subjected to noise, virtual non-enhanced imaging makes it possible to discard the additional acquisition of a pre-contrast scan, dual-energy CT finds application in quantitive assessment of liver lesions and allows for an improved assessment of vascular disease in CT angiography.

A further description of the dual-energy CT is omitted herein, where reference is made to the following publications instead: Thorsten R. C. Johnson et al., Material differentiation by dual energy CT: initial experience, Eur. Radiol.*,DOI* 10.1007/s00330-006-0517-6; S. M. Midgley, A parameterization scheme for the X-ray linear attenuation coefficient and energy absorption coefficient, Phys. Med. Biol. 49 (2004), 307-325; M. Takai and M. Kaneko, Discrimination between thorotrast and iodine contrast medium by means of dual-energy CT scanning, Phy. Med. Biol., No. 8, 29 (1984), 959-967; Y. Ma, N. Li, C. Yang and X. Yang, One-step synthesis of amino-dextran-protected gold and silver nanoparticles and its application in biosensors, Anal Bioanal Chem 382 (2005), 1044-1048; K. Aslan, J. R. Lakowicz and C. D. Geddes, Nanogold-plasmon-resonance-based glucose sensing, Anal. Biochem., 330 (2004), 145-155.

Since the k-edge energy of bismuth lies between the energies of the dual-energy scanning, the X-ray contrast agent can actually be distinguished by the dual-energy scanners. For this reason, the X-ray contrast agent comprising Bi₂O₃-nanoparticles is especially well-suited for dual-energy CT.

In a preferred embodiment, the X-ray contrast agent of the invention is used in addition to a further contrast agent in dual-energy CT, where the further contrast agent can for example be a contrast agent based on iodine. Standard-CT measures only the tissue density in one degree of freedom, therefore pre- and post-contrast CT scans covering the same volume are necessary to measure the concentration of one contrast medium. However, due to the tissue differentiation characteristics of the dual-energy CT modality, it becomes possible to differentiate two contrast media at a time, especially if scans with and without one of the contrast media are performed. In this context, it is desirable to differentiate more than one contrast media at once, especially if those contrast media provide different kinetics. Such a strategy allows to expand the diagnostic potential of the contrast media by providing even more opportunities for characterization of tissues of pathologies, especially in the context of liver lesions or lymph node diagnostics.

For the purposes of promoting and understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated contrast agent and such further applications of the principles of the invention as illustrated therein being contemplated as would normally occur now or in the future to one skilled in the art to which the invention relates.
Fig. 1 is a schematic diagram showing a cross-section of a bismuth oxide nanoparticle covered with a cover layer,
Fig. 2 is a CT angiogram of a mouse obtained with the contrast agent according to an embodiment of the invention, and
Fig. 3 is an X-ray image showing the X-ray absorption of various solutions containing different concentrations of the contrast agent according to an embodiment of the invention in comparison with water and a prior art contrast agent.

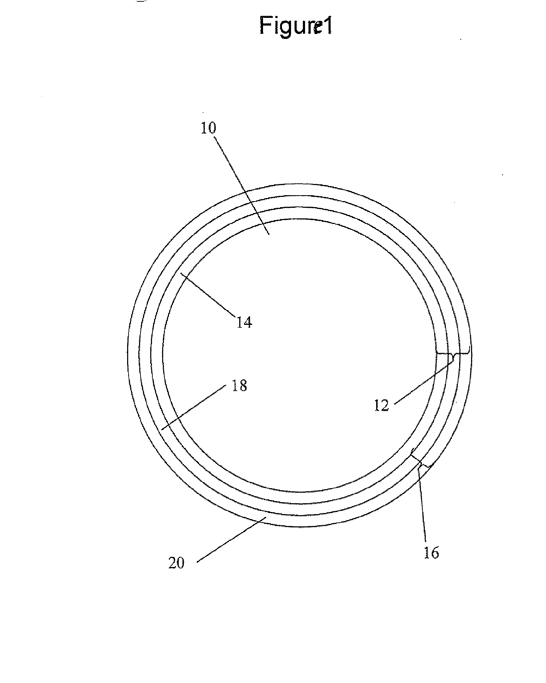

In Fig. 1, a schematic cross-section of a nanoparticle constituting the contrast agent according to an embodiment of the invention is shown. As seen in Fig. 1, the contrast agent is constituted of Bi₂O₃-nanoparticles 10. In the embodiment shown, the nanoparticles 10 have a mean diameter of 62 nm. However, this diameter can vary depending on the intended use or functionality of the contrast agent. The Bi₂O₃-nanoparticle 10 is covered by a cover layer 12, which comprises a stabilizing layer 14 and a functionalizing layer 16. In the shown embodiment, the stabilizing layer 14 is a SiO₂-layer having a thickness of 2 to 4 nm. The functionalizing layer 16 is comprised of silane molecules 18 which are attached to the SiO₂-stabilizing layer 14 and a polymer layer 20, which is attached to the stabilizing layer 14 via the silane molecule layer 18.

The SiO₂-stabilizing layer 14 provides for an inert and stable covering of the Bi₂O₃-nanoparticles and makes the nanoparticles 10 less prone to being solved.

Further, the SiO₂ is well suited for being functionalized by means of a functionalizing layer 16. In the embodiment shown, silane molecules 18 are attached to the SiO₂-layer 14. Preferably, the silane molecule layer 18 is a monomolecular layer, which due to its reactivity allows to attach polymers to the surface of the cover layer 12 for further functionalizing the contrast agent.

Next, a method of making the contrast agent is described.

First, the Bi₂O₃-nanoparticles are made by precipitation from a homogeneous solution of Bi (NO₃)₃ in the presence of a PEG dispersant. The details of making the nanoparticles are known from the Article "Facile synthesis of monodispersant Bi₂O₃-nanoparticles" cited above, which is incorporated herein by reference. In particular, the solution was kept at a temperature of about 90°C and the concentration of the Bi³⁺ was 0.5 mol/l. The reaction time was about 2 hrs.

Using a Zeta-sizer of the Malvern company, it was determined that the mean diameter of the nanoparticles 10 was 62 nm.

Next, the thus formed Bi₂O₃-nanoparticles 10 are covered by a thin layer of SiO₂ using a sol-gel-process. In particular, 1.0 g of Bi₂O₃ is suspended in 65 ml water. By adding 0.8 ml of 25% ammonia and 0.11 ml of 99% acetic acid, the pH-value is adjusted to be 9.0.

Then, 1.67 ml tetraethylorthosilicate solved in 15 ml ethanol are given to the buffered Bi₂O₃ suspension. The suspension is then stirred for 20 hrs. at room temperature. Then, the suspension is subjected to centrifugation and rinsed with water twice. In the actual process, the mean diameter was again measured in a Zeta-sizer and found to be 68 nm, corresponding to a SiO₂-layer of 3 nm.

The SiO₂-layer 14 can then be further functionalized by allowing mono-, di- and/or trialkoxysilanes to react with it. Favourable silanes for this use are 3-amino-propyl-triethoxysilane (APTES) or 3-mercaptopropyl-triethoxysilane. Preferably, the binding of the silane molecules with the SiO₂-layer 14 is performed in the same reaction solution that was used when forming the SiO₂-layer. This can be done by dripping the silane solved in ethanol to the suspension of SiO₂-covered nanoparticles under reflow.

In a preferred embodiment, the suspension obtained when forming the SiO₂-layer is heated in an oil bath under backflow until boiling. Then, while maintaining the heating, a dropping funnel is used to slowly add 0.15 g of 3-aminopropyltriethoxysilane solved in 20 ml ethanol. The suspension is boiled for a total of 3 hrs. under reflow. Then the particles are subjected to centrifugation and washed with ethanol once and twice with water.

Then, the thus prepared particles can be functionalized using dextrane or another polar polymer which binds with the silane molecules.

With this method, a very stable new contrast agent is obtained which allows for angiographic or gastroenterologic examinations without toxic adverse effects. In fact, the new contrast agent is found to have little or no nephrotoxicity and does not adversely affect the thyroid hormone processes. Due to the high electron density of the bismuth contrast agent, it provides high X-ray absorption and thus allows for enhanced contrast in the X-ray images.

Further, by varying the generic process described above, the nanoparticle size and the surface of the particles can be varied, such that the resulting contrast agent will be specifically adapted for certain tissues or metabolic pathways. This way, the contrast agent can for example be specifically adapted for imaging of the liver, lymph nodes and the reticulo-endothelial system. For example, the size of the Bi₂O₃-nanoparticles 10 can be easily and reproducibly controlled by suitable control of the reaction temperature, reaction time, reactant and dispersant concentration. Also, the Bi₂O₃-nanoparticles 10 achieved by the above described method have found to be very stable over a period of several months.

By proper choice of silane molecules and/or polymers constituting the functionalizing layer 16, the contrast agent can be functionalized such as to access pre-determined metabolic pathways and to thus allow for functional imaging.

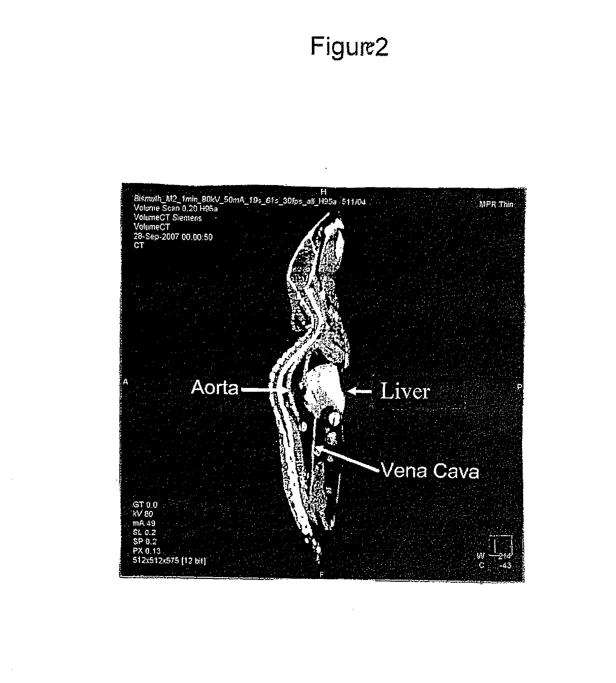

Fig. 2 shows a CT-angiography of a mouse using bismuth oxide-nanoparticle contrast agent according to an embodiment of the invention. The scan has been made shortly after injection of a suspension of bismuth oxide-nanoparticles. As can be seen in Fig. 2, the vena cava, the liver and the aorta show a clear contrast.

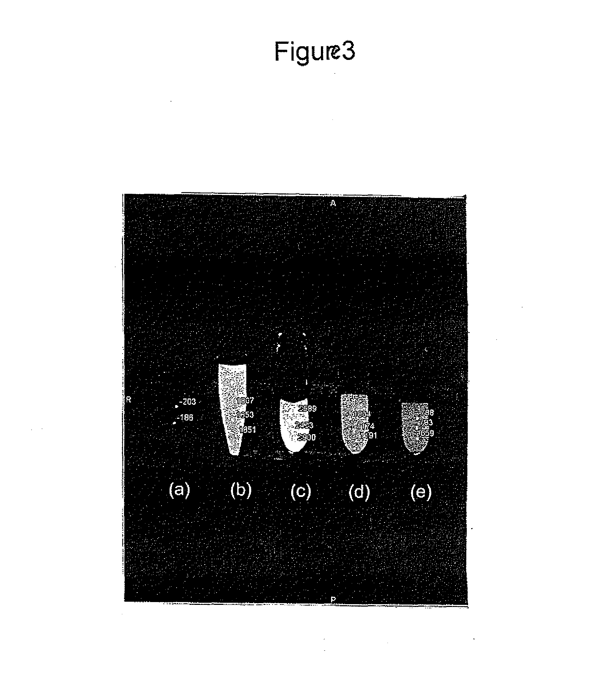

In Fig. 3, the contrast obtainable with bismuth oxide-nanoparticles as compared to prior art contrast agent Imeron 100 is shown. In the X-ray image of Fig. 3, panel (a) represents water, panel (b) represents Imeron 100, and panels (c), (d), and (e) correspond to bismuth oxide-nanoparticles at concentrations of 0.96 mol/l, 0.32 mol/l and 0.24 mol/l, respectively. At a concentration of 0.96 mol/l, i.e. panel (c) of Fig. 3, an X-ray absorption is found that exceeds the X-ray absorption of Imeron 100.

Although a preferred exemplary embodiment is shown and specified in detail in the preceding specification, this should be viewed as purely exemplary and not as limiting the invention. It is noted in this regard that only the preferred exemplary embodiment is shown and specified, and all variations and modifications should be protected that presently or in the future lie within the scope of protection of the invention as defined in the claims.

### Reference list:

- 10: Bi₂O₃-nanoparticle
- 12: cover layer
- 14: stabilizing layer
- 16: functionalizing layer
- 18: silane molecules
- 20: polymer layer

## Claims

1. An X-ray contrast agent comprising Bi₂O₃-nanoparticles (10) covered with a cover layer (12) for stabilizing and/or functionalizing the contrast agent.

2. The X-ray contrast agent according to claim 1, wherein the diameter of the Bi₂O₃-nanoparticles is larger than 20 nm, preferably larger than 30 nm and most preferably larger than 40 nm.

3. The X-ray contrast agent according to claim 1 or 2, wherein the cover layer (12) comprises a stabilizing layer (14) for increasing the inertness and decreasing the solubility of the Bi₂O₃-nanoparticle (10), and
a functionalizing layer (16) formed on top of the stabilizing layer (14) for functionalizing the contrast agent.

4. The X-ray contrast agent according to one of the preceding claims, wherein the stabilizing layer (14) comprises SiO₂.

5. X-ray contrast agent according to one of the preceding claims, wherein the functionalizing layer (16) comprises silane molecules (18) bound to the stabilizing layer (14), and in particular, mono-, di-, and/or trialkoxysilane molecules.

6. The X-ray contrast agent according to one of claims 3 to 5, wherein the functionalizing layer (16) comprises polar polymers (20) attached to the stabilizing layer (14).

7. A set of X-ray contrast agents,
wherein each of the contrast agents is a contrast agent as defined in one of claims 1 to 6, and
wherein each of the individual contrast agents contained in the set is specified for use for one or a group of different target areas or metabolic pathways,
said specification being based on the size of the nanoparticles (10) and/or the constitution of their cover layers (12).

8. A method of making an X-ray contrast agent, said method comprising a Bi₂O₃-nanoparticle forming step of forming Bi₂O₃-nanoparticles (10), and
a covering step of forming a cover layer (12) on top of the Bi₂O₃-nanoparticles (10), said cover layer (12) being suitable for stabilizing and/or functionalizing the contrast agent,

9. The method of claim 8, wherein the nanoparticle forming step comprises a step of precipitating the Bi₂O₃-nanoparticles from a Bi (NO₃)₃-solution in the presence of a dispersant, in particular a PEG-dispersant.

10. The method of claim 8 or 9, wherein the covering step comprises a step of growing an inert layer (14) on top of the Bi₂O₃-nanoparticle (10), and in particular, a SiO₂-layer.

11. The method of claim 10, wherein the step of growing an inert layer (14) comprises a step of forming a SiO₂-layer on top of the Bi₂O₃-nanoparticle (10) by suspending Bi₂O₃-nanoparticles (10) in an aqueous solution having a pH of 8.5 to 9.5 and adding a solution containing tetraethylorthosilicate.

12. The method of claim 10 or 11, wherein the covering step comprises a step of causing silane molecules to bind with the surface of the inert layer (14).

13. The method of claims 11 and 12, wherein the silane molecules are caused to bind with the SiO₂-layer (14) by adding silane molecules to the same solution in which the SiO₂-layer (14) has been grown.

14. The method of claims 8 to 14, wherein the covering step further comprises a step of binding a polar polymer to the cover layer (12).

15. Use of an X-ray contrast agent according to one of claims 1 to 6 in dual-energy CT.
